# EUROPEAN PATENT APPLICATION

(11) **EP 4 643 656 A2**
(43) Date of publication of application: **05.11.2025**
(21) Application number: 25204001.9
(22) Date of filing: 07.12.2021
(51) Int. Cl.: A23L 33/10

(54) **IMPROVED PREBIOTIC FORMULATIONS**

(30) Priority: 11.12.2020 US 202063124384 P
(62) Divisional of application: 21834754.0
(71) Applicant: DSM IP Assets B.V., 6221 BE Maastricht (NL)
(72) Inventor: VAN LEEUWEN, Johannes Gustaaf Ernst, 6100 AA ECHT (NL); WIENS, Frank., 6100 AA ECHT (NL); VAN DEN BERG, Marco Alexander., 6100 AA ECHT (NL)
(74) Representative: dsm-firmenich IP

(57) **Abstract**

Process comprising reducing a fermentation broth comprising 2'-fucosyllactose to produce a treated fermentation broth comprising 2'-fucosyllactose and 2'-fucosyllactitol; isolating a stream comprising 2'-fucosyllactose and 2'-fucosyllactitol from the treated fermentation broth; purifying the isolated stream; concentrating the purified isolated stream; dewatering the concentrated purified isolated stream, thereby forming a prebiotic solids product comprising 2'-fucosyllactose and 0.2 to 6.0 wt.% of 2'-fucosyllactitol based on the total wt.% of the prebiotic solids product, wherein the concentrating step and dewatering step can be performed in any order.

## Description

### FIELD OF THE INVENTION

The invention relates to improved prebiotic formulations. More particularly, the invention relates to prebiotic formulations containing 2'-fucosyllactose and 2'-fucosyllactitol.

### BACKGROUND OF THE INVENTION

Human milk contains a family of unique oligosaccharides, known as human milk oligosaccharides (HMOs), which are structurally diverse unconjugated glycans. Despite being the third most abundant solid component of human milk (after lactose and fat), human infants cannot digest HMOs. Instead, they function as prebiotics to help establish commensal bacteria. Prebiotics or non-digestible carbohydrates are used to enhance specific and beneficial microbial species. For example, 2'-fucosyllactose (2'-FL), naturally present in human breast milk, is a known prebiotic stimulating the growth of specific and beneficial microbiota of the genus Bifidobacterium. 2'-FL is indigestible by human endogenous enzymes but is used by mutualist symbiotic bacteria in the infant gut as an energy source to selectively support beneficial bacterial growth (prebiotic effect). It is secreted into the milk of lactating women from their mammary gland epithelial cells together with potentially > 200 similar human oligosaccharides. The α1,2/3/4 fucosyl moieties of HMOs, including the α1,2-linked fucose of 2'-FL, tend to shield HMOs from digestion unless these linkages at the non-reducing terminus are first cleaved. Therefore, prebiotic effects of 2'-FL depend on several activities by bacterial enzymes, such as α1,2-fucosidase activity that cleave of the fucose and release lactose; activities that metabolize the released fucose into lactate and 1,2-propanediol; activities that metabolize the proportion of lactose, which is not used by human host cells, and some other activities. Supporting the development of a healthy digestive tract in infants assists in the development of their immune systems since much of the infant's immune system is in the digestive tract. The occurrence and concentration of these complex oligosaccharides are specific to humans and are not found in large quantities in the milk of other mammals such as domesticated dairy animals. Work has been ongoing to develop improved systems for producing HMOs, as in EP 14827224.8, WO2019/003133, WO2019/003135, U.S. 2017/0304375, and WO2019/003136.

Thus, it is known that a well-balanced microbiota composition is beneficial for general health and well-being. Lactitol is a known prebiotic, enhancing microbes of the species Lactobacillus (and to a lesser extent Bifidobacterium) in the gut, as well as increasing the levels of short chain fatty acids. A composition comprising 2'-fucosyllactitol (2'-FLol) will stimulate a broad range of beneficial microbiota and thereby lead to a better-balanced gut, since 2'-FLol is readily hydrolyzed by specific fucosidases produced by gut microbiota (Bifidobacterium), releasing lactitol and fucose. In addition, lactitol is not digested by human digestive enzymes, while lactose is. Thus, mixtures of 2'-FL and 2'-FLol are believed to provide a more robust and versatile prebiotic system than 2'-FL alone since their effects are complimentary; for example, should 2'-FL become hydrolyzed too soon during storage or in meal preparation, in the mixed system 2'-FLol or lactitol is still present. Moreover, lactitol has a caloric value of 2 kcal/g, while lactose has a caloric value of 3.94 kcal/g.

2'-FL is typically produced in microbial fermentation reactors; however, such conventional processes produce only low levels of 2'-FLol as a byproduct. A need exists for a process to produce compositions containing both 2'-FL and 2'-FLol where 2'-FLol is present at increased levels.

### SUMMARY OF THE INVENTION

A process for producing mixtures containing both 2'-FL and 2'-FLol, with 2'-FLol present at increased levels has unexpectedly been discovered, where 2'-FL is produced in a microbial fermentation which is subjected to reducing conditions to produce 2'-FLol.

In one embodiment, the present disclosure provides a process comprising reducing a fermentation broth comprising 2'-fucosyllactose to produce a treated fermentation broth comprising 2'-fucosyllactose and 2'-fucosyllactitol; isolating a stream comprising 2'- fucosyllactose and 2'-fucosyllactitol from the treated fermentation broth; purifying the isolated stream; concentrating the purified, isolated stream; dewatering the concentrated purified, isolated stream, thereby forming a prebiotic solids product comprising 2'-fucosyllactose and 0.2 to 6.0 wt% of 2'-fucosyllactitol based on the total wt% of the prebiotic solids product, wherein the concentrating step and dewatering step can be performed in any order.

In another embodiment, the present disclosure provides a process comprising administering an effective amount of the prebiotic solids product composition to a subject in need thereof, thereby generating an *in vivo* supply of lactitol.

In still another embodiment, the present disclosure provides a process for making a food, growing up milk, dietary supplement or medicine, wherein the process comprises: making the prebiotic solids product; and mixing the prebiotic solids product with one or more ingredients suitable for the food, growing up milk, dietary supplement or medicine.

In another embodiment, the present disclosure provides a process for making a food, growing up milk, dietary supplement or medicine, wherein the process comprises making the prebiotic solids product; dissolving the prebiotic solids product in a solvent; and mixing the dissolved prebiotic solids product with one or more ingredients suitable for the food, growing up milk, dietary supplement or medicine.

In still another embodiment, the present disclosure provides a process for making infant formula, wherein the process comprises making the prebiotic solids product and mixing the prebiotic solids product with at least one infant formula ingredient.

In an embodiment, the present disclosure provides a process for making infant formula, wherein the process comprises making the prebiotic solids product and dissolving the prebiotic solids product in a solvent; and mixing the dissolved prebiotic solids product with at least one infant formula ingredient.

In another embodiment, the present disclosure provides a food, growing up milk, dietary supplement or medicine obtained from a process wherein the process comprises making the prebiotic solids product and either mixing the prebiotic solids product with at least one infant formula ingredient, or first dissolving the prebiotic solids product in a solvent; and mixing the dissolved prebiotic solids product with at least one infant formula ingredient.

### BRIEF DESCRIPTION OF THE DRAWINGS

The subject matter of the present disclosure will be more fully understood from the following detailed description, taken in connection with the accompanying drawings, in which:

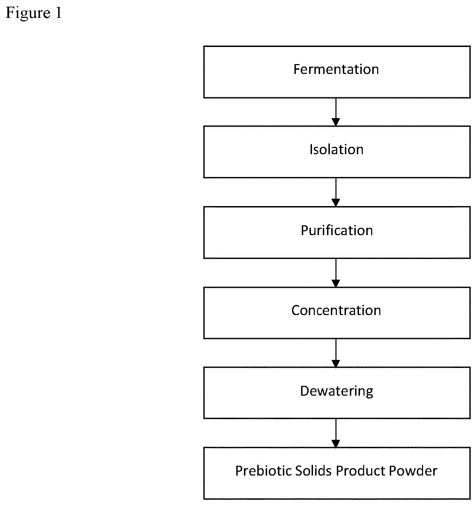

Figure 1 is a process flow diagram describing the separation and purification of a prebiotic solids stream.

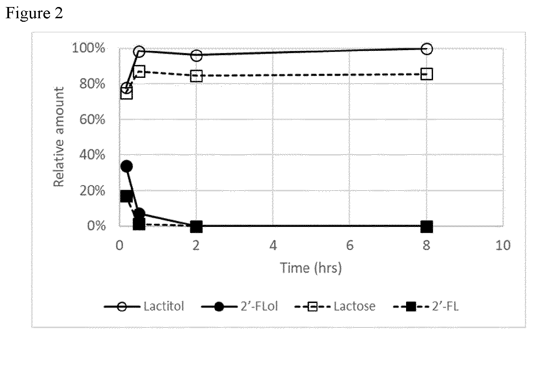

Figure 2 illustrates hydrolysis of composition 2 (47mM 2'-FL and 2mM 2'-FLol) by 12 µg/ml fucosidase from *Bifidobacterium longum,* to lactose and lactitol respectively, in 125 mM sodium phosphate buffer pH 6.5 at 37°C. Solid lines, 2'-FLol (closed circles) and its derived product lactitol (open circles); dashed lines, 2'-FL (closed squares) and its derived product lactose (open squares).

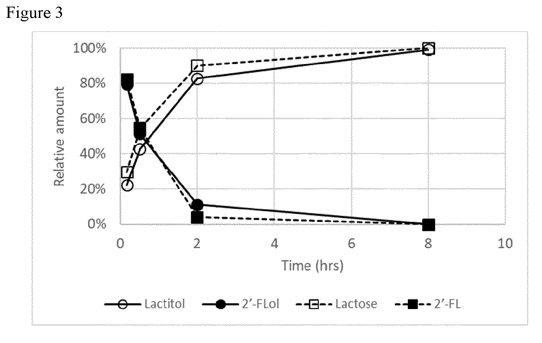

Figure 3 illustrates hydrolysis of composition 2 (47mM 2'-FL and 2mM 2'-FLol) by 12 µg/ml fucosidase from *Bifidobacterium sp.,* to lactose and lactitol respectively, in 125 mM sodium phosphate buffer pH 6.5 at 37°C. Solid lines, 2'-FLol (closed circles) and its derived product lactitol (open circles); dashed lines, 2'-FL (closed squares) and its derived product lactose (open squares).

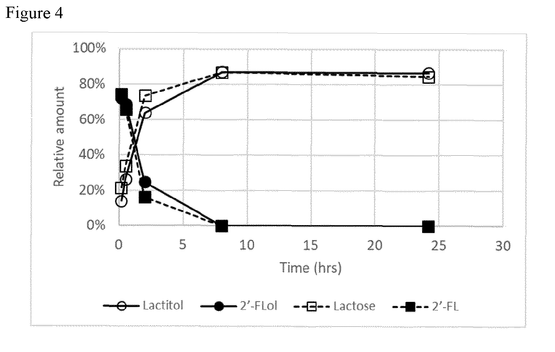

Figure 4 illustrates hydrolysis of composition 2 (47mM 2'-FL and 2mM 2'-FLol) by 2 µg/ml fucosidase from *Bifidobacterium longum,* to lactose and lactitol respectively, in 125 mM sodium phosphate buffer pH 6.5 at 37°C. Solid lines, 2'-FLol (closed circles) and its derived product lactitol (open circles); dashed lines, 2'-FL (closed squares) and its derived product lactose (open squares).

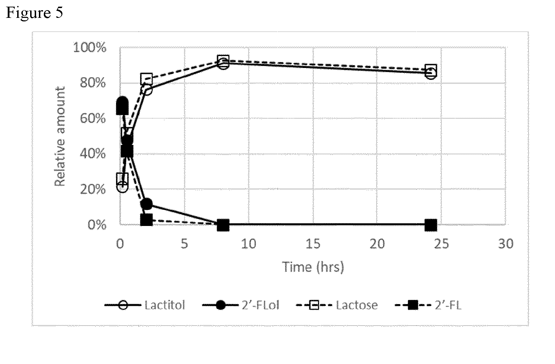

Figure 5 illustrates hydrolysis of composition 2 (47mM 2'-FL and 2mM 2'-FLol) by 2 µg/ml fucosidase from *Bifidobacterium sp.,* to lactose and lactitol respectively, in 125 mM sodium phosphate buffer pH 6.5 at 37°C. Solid lines, 2'-FLol (closed circles) and its derived product lactitol (open circles); dashed lines, 2'-FL (closed squares) and its derived product lactose (open squares).

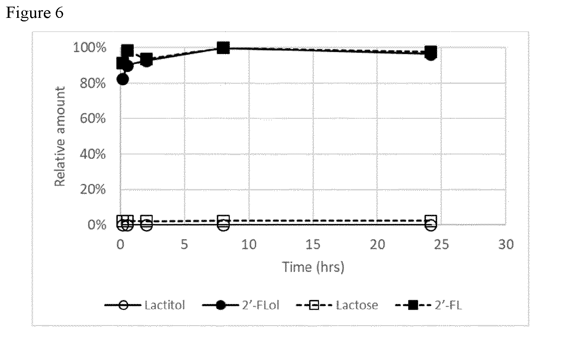

Figure 6 illustrates no-enzyme control incubation of composition 2 (47mM 2'-FL and 2mM 2'-FLol) with water, in 125 mM sodium phosphate buffer pH 6.5 at 37°C. Solid lines, 2'-FLol (closed circles) and its derived product lactitol (open circles); dashed lines, 2'-FL (closed squares) and its derived product lactose (open squares).

### DETAILED DESCRIPTION OF THE INVENTION

The present disclosure features methods of producing, separating, and purifying an improved prebiotic solids product. In one embodiment, the present disclosure provides a process comprising reducing a fermentation broth comprising 2'-FL to produce a treated fermentation broth comprising 2'-FL and 2'FLol; isolating a stream comprising 2'-FL and 2'-FLol from the reduced fermentation broth; purifying the isolated stream; concentrating the purified isolated stream; dewatering the concentrated purified isolated stream thereby forming a prebiotic solids product comprising 2'-fucosyllactose and 0.2 to 6.0 wt.% of 2'-fucosyllactitol based on the total wt.% of the prebiotic solids product, wherein the concentrating step and dewatering step can be performed in any order. For the purposes of this specification, the term prebiotic means a selectively fermented ingredient that results in specific changes in the composition and/or activity of the gastrointestinal microbiota, thus conferring benefit(s) upon the host's health. Characteristics of the prebiotic include: (i) it should be resistant to the acidic pH of the stomach, (ii) it cannot be hydrolyzed by mammalian enzymes, (iii) it should not be absorbed in the gastrointestinal tract, (iv) it can be fermented by intestinal microbiota, and (iii) the growth and/or activity of the intestinal bacteria can be selectively stimulated by this compound, and this process improves the host's health.

### Fermentation Step

Fermentation may be performed in any suitable fermentation medium, such as, for example, a chemically defined fermentation medium. The fermentation medium may vary based on the microbial organism used.

Preferably, in the fermentation step, sugar, lactose and fermentation nutrients are fed to a culture of a microbe genetically modified to produce 2'-FL, and then fermented to produce 2'-FL and other carbohydrates such as difucosyllactose, 2'-fucosyl-d-lactulose, lactose/allo-lactose, 3'-fucosyllactose, fucosyl-galactose. The fermentation medium can be supplemented with trace minerals and vitamins such as calcium panththenate, biotin, nicotinic acid, myo-inositol, thiamine HCL, p-aminobenzoic acid. Food grade processing aids such as antifoam and pH control agents can also be used. The temperature of the fermentation step is typically 10 to 50°C, preferably 25 to 35°C, more preferably 28 to 32°C. The pH of the fermentation step is typically 3 to 8, preferably, 5 to 7, more preferably 5.5 to 6.5. At the conclusion of the fermentation step, the fermentation broth can be subjected to a heat treatment step to deactivate the microbe prior to the downstream treatment steps.

Preferably, the microbial organism is a genetically modified yeast such as a *Saccharomyces* strain, a *Candida* strain, a *Hansenula* strain, a *Kluyveromyces* strain, a *Pichia* strain, a *Schizosaccharomyces* stain, a *Schwanniomyces* strain, a *Torulaspora* strain, a *Yarrowia* strain, or a *Zygosaccharomyces* strain. More preferably, the yeast is *Saccharomyces cerevisiae, Hansenula polymorpha, Kluyveromyces lactis, Kluyveromyces marxianus, Pichia pastoris, Pichia methanolica, Pichia stipites, Candida boidinii, Schizosaccharomyces **pombe**, Schwanniomyces occidentalis, Torulaspora delbrueckii, Yarrowia lipolytica, Zygosaccharomyces rouxii,* or *Zygosaccharomyces bailii.*

Preferably, the microbial organism can also be selected from the bacterial genera Bifidobacterium, Lactobacillus, Enterococcus, Streptococcus, Staphylococcus, Peptostreptococcus, Leuconostoc, Clostridium, Eubacterium, Veilonella, Fusobacterium, Bacterioides, Prevotella, Escherichia, or Propionibacterium. More preferably, the bacterial species is Bifidobacterium adolescentis, B. animalis, B. bifidum, B. breve, B. infantis, B. lactis, B. longum; Enterococcus faecium; Escherichia coli,; Lactobacillus acidophilus, L. bulgaricus, L. casei, L. crispatus, L. fermentum, L. gasseri, L. helveticus, L. johnsonii, L. paracasei, L. plantarum, L. reuteri, L. rhamnosus, L. salivarius, L. sakel, Lactococcus lactis (including but not limited to the subspecies lactis, cremoris and diacetylactis); Leuconostoc mesenteroides (including but not limited to subspecies mesenteroides); Pedicoccus acidilactici, P. pentosaceus; Propionibacterium acidipropionici, P. freudenreichii ssp. shermanii; Staphylococcus carnosus; and Streptococcus thermophilus. The fermentation broth can also include other products of the fermentation process such as cells (biomass), the fermentation medium, residual substrate material, and any molecules/by-products produced during fermentation.

Optionally, the fermentation broth can be subjected to a heat treatment step to kill bacteria or other undesired micro-organisms that may be present to any significant extent.

### Reduction Step

The fermentation broth comprising 2'-fucosyllactose is reduced to produce a treated fermentation broth comprising 2'-fucosyllactose and 2'-fucosyllactitol. During this reduction, 2'- fucosyllactitol is formed through at least one of two pathways:
Pathway #1
   - Lactose + 2 H⁺ + 2 e⁻ **→** Lactitol
   - Lactitol + GDP-fucose **→** 2'-fucosyllactitol + GDP
Pathway #2
   - Lactose + GDP-fucose **→** 2'-fucosyllactose + GDP
   - 2'-fucosyllactose + 2 H⁺ + 2 e⁻ **→** 2'-fucosyllactitol

In these pathways, the term "2 H⁺ + 2 e⁻" represents reduction equivalents, typically present as NAD(H), NADP(H), FAD(H) or intermediates. In both pathways, the reactions are catalyzed by microbial enzymes, and these reactions would occur inside the microbe. The pathways involve reduction reactions, and thus require reduction equivalents (H⁺ + e⁻). The extent of 2'-fucosyllactitol production can be affected by oxygen flow, where decreases in oxygen concentration enhance reducing conditions; the relative flow of lactose, which is reduced directly to lactitol in Pathway #1; the relative flow of sucrose, which serves as a carbon source and electron carrier; or by variations in 2'-fucosyllactose export from the microbial cell. Alternatively, the reduction occurs spontaneously during the fermentation process. Temperature and pressures for the reduction step are as those for the fermentation step.

After fermentation and reduction of the fermentation broth, the product of those steps is subjected to a variety of steps to isolate and purify a dry mixture of 2'-FL and 2'-FLol. Such steps are illustrated in Figure 1. An isolation step is used to separate the 2'-FL and 2'- FLol from other materials contained in the fermentation broth. Such an isolation step can include centrifugation, conventional filtration, as described below, and microfiltration, which produces an isolated stream rich in 2'-FL and 2'-FLol. Following the isolation step, the isolated stream is subjected to a purification step which can include ultrafiltration, nanofiltration, anion exchange, cation exchange and decolorization, to form a purified, isolated stream. The purified, isolated stream is then treated in a concentration step and a dewatering step. These steps can be performed in any order. In other words, the purified, isolated stream can first be treated in a concentration step, and the product of the concentration then routed to a dewatering step. Alternately, the purified, isolated stream can first be subjected to a dewatering step, and the product of that dewatering step can then be routed to a concentration step. The concentration step can include evaporation, reverse-osmosis filtration and nanofiltration. Evaporation processes can include, e.g., falling film evaporation, climbing film evaporation and rotary evaporation, to form a concentrated, purified, isolated stream. The dewatering step can include at least one of crystallization, drying, evaporation and conventional filtration, to form a prebiotic solids product.

The various unit operations utilized in the separation/purification process steps of the present disclosure are described below. Combinations of these unit operations can be utilized depending upon the purity levels that are desired. It is possible that some unit operations, such as centrifugation and conventional filtration can also be utilized in both the isolation and concentration steps.

### Operations for separation and purification of fermentation broth

### Conventional Filtration

For the purpose of this specification, the term conventional filtration refers to processes using plate and frame filtration, recessed chamber filtration, belt filtration, vacuum filtration, horizontal metal leaf filtration, vertical metal-leaf filtration, stacked-disc filtration, rotary vacuum filtration and combinations thereof.

### Centrifugation

Centrifugation can be used to remove suspended matter such as biomass. The desired prebiotic solids product is contained in the liquid not retained by the centrifuge. Preferably, the centrifuge is operated continuously.

### Microfiltration

A cross flow filtration process can be used to serve as a guard filter to remove residual biomass that is not removed in the centrifugation step. Typically, the microfiltration has a cut off of 10 microns, preferably a cut off of 5 microns, more preferably a cut off of 0.2 microns. The product stream is the liquid permeate.

### Ultrafiltration

A cross flow ultrafiltration can be used to remove proteins and other high molecular weight compounds, such as DNA and large polysaccharides from the fermentation product. The pore size of the ultrafiltration membrane ranges from about a 200 kD molecular weight cut-off ("MWCO") or less to about 1 kD MWCO. The product stream is the permeate. **[0037]** Preferably, the yield of the desired prebiotic solids in the permeate after an ultrafiltration step is greater than 50%, greater than 60%, greater than 70%, greater than 80%, greater than 90%, greater than 91%, greater than 92%, greater than 93%, greater than 94%, greater than 95%, greater
than 96%, greater than 97%, greater than 98%, or greater than 99%.

### Nanofiltration

Cross flow nanofiltration can be used to remove low molecular weight molecules smaller than the desired prebiotic solids, such as mono- and disaccharides, peptides, small organic acids, and salts. The pore size of the nanofiltration membrane is from about 500 dalton (Da) or less molecular weight cut-off to 200 Da MWCO or less. Preferably, 500 dalton (Da) or less molecular weight cut-off, 450 Da MWCO, 400 Da MWCO, 350 Da MWCO, 300 Da MWCO, 250 Da MWCO, or 200 Da MWCO or less. The product stream is the retentate.

Preferably, the yield of the desired prebiotic solids in the retentate after a nanofiltration step is greater than 50%, greater than 60%, greater than 70%, greater than 80%, greater than 90%, greater than 91%, greater than 92%, greater than 93%, greater than 94%, greater than 95%, greater
than 96%, greater than 97%, greater than 98%, or greater than 99%.

### Cation Exchange (CEX)

Ion exchange chromatography can be used to separate charged molecules from the prebiotic solids-containing stream. The CEX stationary phase usually contains aliphatic sulfonic acid groups that are negatively charged in aqueous solution, which tightly bind any strongly basic analytes. This step removes positively charged components, e.g., residual ammonia, metals and peptides. The binding capacity of the resins used are generally 1.2 to 2.2 mmeq/L. Typical resins used for cation exchange include Dow Dowex 88, Resindion JC series such as JC603, and Resindion PK grades such as PK216.

### Decolorization

A decolorization step can be performed to remove color-containing components. This step can be conducted using activated carbon, such as Norit CA1 activated carbon, a Hydrophobic Interaction Chromatography (HIC), or another adsorptive resin which can be functionalized, such as Resindion Relite RAD/F. The decolorization can be performed either before or after the anion exchange step.

### Anion Exchange

An anion exchange step can be used to separate charged molecules. In an anion exchange column, the packing is positively charged, and therefore retains negatively charged molecules by coulombic interaction. This step removes negatively charged components, e.g., sulphates, phosphates, organic acids, and negatively charged particles. The binding capacity of the resins used are generally 0.8 to 2.0 mmeq/L. Typical resins for anion exchange include Resindion Relite series such as RAM2, D182, and JA100, and Diaion WA series such as WA20.

### Concentration

A concentration step can be used to economically remove significant quantities of liquid from the prebiotic solids-containing stream using evaporation, reverse-osmosis filtration and nanofiltration. Evaporation processes can include, e.g., falling film evaporation, climbing film evaporation and rotary evaporation. The incoming solids concentration to the process is approximately 5 to 30 wt.%. The exit solids concentration from such a process is typically greater than 30 wt.%., preferably greater than 50 wt.%. More preferably, the solids concentration exiting the dewatering operation is 60 to 80 wt.%. The solids portion of the recovered material is greater than 80 wt.% prebiotic solids, with the remaining impurities primarily being alditols or di- or trisaccharides.

### Heat Treatment

A heat treatment step can be used to kill bacteria or other undesired micro-organisms that may be present to any significant extent and is basically a pasteurization process. Any acceptable pasteurization conditions are possible, e.g., from 62.8°C for 30 minutes to 72°C for 20 seconds to 100°C for 0.01 seconds.

### Drying

Drying may be conducted by indirect dryers in indirect drying processes or by direct dryers in direct drying processes. For the purposes of this specification, the term indirect dryer includes those devices that do not utilize direct contact of the material to be dried with a heated process gas for drying, but instead rely on heat transfer either through walls of the dryer, e.g., through the shell walls in the case of a drum dryer, or alternately through the walls of hollow paddles of a paddle dryer, as they rotate through the solids while the heat transfer medium circulates in the hollow interior of the paddles. Other examples of indirect dryers include contact dryers and vacuum drum dryers. The heat transfer medium is preferably steam or heat transfer oils. More preferably, the heat transfer medium is steam. The term direct dryers include processes where a hot process gas moves through the vessel, directly contacting the circulating solids, such as spray dryers. Flash dryers or fluid bed dryers are additional examples of such direct drying methods. Preferably when a direct drying is used it is conducted by spray drying in a spray dryer. Preferably, the drying method is an indirect drying. Preferably, the indirect drying method is drum drying. In drum drying, the heated surface is the envelope of a rotating horizontal metal cylinder. The cylinder is preferably heated by steam condensing inside, bringing the temperature of the cylinder wall to 110-155°C. Preferably, the prebiotic solids material has a residence time on the dryer of less than 3 minutes. The wet material is applied onto the drum surface as a relatively thin layer. The dried product is removed from the drum
with the assistance of a blade or strings. Subsequently, a milling and sieving step may be used to obtain the desired particle size range.

### Milling

If a milling step is required, generally any milling method suitable for the type of solids and particle sizes targeted can be used. Such milling equipment can include, e.g., ball mills, hammer mills, SAG mills, rod mills, Raymond mills and vertical mills.

### Crystallization

A crystallization process can be used to dewater the purified prebiotics solids material by first forming prebiotic solids nuclei in a supersaturated solution and then growing the crystals. Such a process can be either batch or continuous. Typical conditions used for the crystallization are shown in WO 2018/164937, the disclosure of which is incorporated herein by reference. The process involves concentrating a starting prebiotic solids-containing solution to a supersaturated state, and then precipitating the prebiotic solids crystal from the supersaturated solution while preferably subjecting the supersaturated solution to a temperature of at least 55°C. Preferably, the solution has a prebiotic solids content of at least 60 wt.% and less than about 98 wt.%. Crystallization is typically limited by viscosity as the percentage of the prebiotic solid crystals grows, preferably, with a typical endpoint of around 30 wt.% crystals.

### Prebiotic Solids Product

The prebiotic solids product contains 0.2 to 6.0 wt.% 2'-FLol, 86.0 to 94.0 wt.% 2'-FL, and 0 to 13.8 wt.% carbohydrate byproducts. For the purposes of this specification, the term "carbohydrate byproducts" includes difucosyllactose, 2'-fucosyl-d-lactulose, lactose/allo-lactose, 3'-fucosyllactose, fucosyl-galactose, glucose/galactose, and fructose. Quantities of these materials are determined using ion chromatography pulse amperometry detection with a Dionex Carbopac PA1 column.

Preferably, the prebiotic solids contain 0.5 to 6.0 wt.%, more preferably 0.5 to 3.5 wt.% 2'-FLol, with corresponding levels of other carbohydrates being 0 to 13.5 wt.% and 2.5 to 13.5 wt.%, respectively. In both cases, 2'-FL is present in an amount of 86.0 to 94.0 wt.%.

In another embodiment, the prebiotic solids contain 2.0 to 4.5 wt.%, more preferably 2.0 to 4.0 wt.% 2'-FLol, with corresponding levels of carbohydrate byproducts being 1.5 to 12.0 wt.% and 2.0 to 12.0 wt.%, respectively. In both cases 2'-FL is present in an amount of 86.0 to 94.0 area.
In still another embodiment, the prebiotic solids contain 0.2 to 10.0 wt.% 2'-FLol, 86.0 to 90.0 wt.% 2'-FL, and 0 to 13.8 wt.% carbohydrate byproducts.

Preferably, the ratio of 2'-FL/2'-FLol is 14.3 to 470.

In another embodiment, the present disclosure provides a process comprising administering an effective amount of the prebiotic solids product composition to a subject in need thereof, thereby generating an *in vivo* supply of lactitol. As discussed above, in the gut 2'-FLol is hydrolyzed to fucose and lactitol.

In another embodiment, the present disclosure provides a process for making a food, growing up milk, dietary supplement or medicine, wherein the process comprises: making the prebiotic solids product; and mixing the prebiotic solids product with one or more ingredients suitable for the food, growing up milk, dietary supplement or medicine.

In still another embodiment, the present disclosure provides a process for making a food, growing up milk, dietary supplement or medicine, wherein the process comprises making the prebiotic solids product; dissolving the prebiotic solids product in a solvent; and mixing the dissolved prebiotic solids product with one or more ingredients suitable for the food, growing up milk, dietary supplement or medicine.

In another embodiment, the present disclosure provides a process for making infant formula, wherein the process comprises making the prebiotic solids product and mixing the prebiotic solids product with at least one infant formula ingredient.

In an embodiment, the present disclosure provides a process for making infant formula, wherein the process comprises making the prebiotic solids product and dissolving the prebiotic solids product in a solvent; and mixing the dissolved prebiotic solids product with at least one infant formula ingredient.

In another embodiment, the present disclosure provides a food, growing up milk, dietary supplement or medicine obtained from a process wherein the process comprises making the prebiotic solids product and either mixing the prebiotic solids product with at least one infant formula ingredient, or first dissolving the prebiotic solids product in a solvent; and mixing the dissolved prebiotic solids product with at least one infant formula ingredient.

For the purpose of this specification, the term "Infant Formula" means a breast milk substitute formulated industrially in accordance with applicable Codex Alimentarius and United

States Food and Drug Administration standards (i) to satisfy the total normal nutritional requirements of infants from birth up to between four and six months of age and adapted to their physiological characteristics and/or fed in addition to other foods to infants up to approximately one year of age and older or (ii) to satisfy the normal nutritional requirements of infants born prematurely. The term "infant" means a child from birth up to approximately one year of age. The term "Growing-up Milks (GUMs)" means a nutritionally enhanced milk, plant, amino acid and/or soy-milk based liquid products, or powdered products for reconstitution as liquid products, in each case intended for use as a beverage, and marketed to and intended for use by children from twelve (12) months through thirty-six (36) months, and for children up to and including six (6) years of age.

Many different sources and types of carbohydrates, fats, proteins, minerals and vitamins are known and can be used as formula ingredients in the infant formulas or GUMs described in the current subject matter, provided that such nutrients are compatible with the added ingredients in the selected formulation and are otherwise suitable for use in an infant formula.

Carbohydrates suitable for use in the infant formulas may be simple or complex, lactose-containing or lactose-free, or combinations thereof, non-limiting examples of which include hydrolyzed, intact, naturally and/or chemically modified cornstarch, maltodextrin, glucose polymers, sucrose, corn syrup, corn syrup solids, rice or potato derived carbohydrate, glucose, fructose, lactose, high fructose corn syrup and indigestible oligosaccharides such as 3'-fucosyllactose, 2',3'-difucosyllactose, lacto-N-triose II, lacto-N-tetraose, lacto-N-neotetraose, lacto-N- fucopentaose I, lacto-N-neofucopentaose, lacto-N-fucopentaose II, lacto-N- fucopentaose Ill, lacto-N-fucopentaose V, lacto-N-neofucopentaose V, lacto-N- difucohexaose I, lacto-N-difucohexaose II, 6'-galactosyllactose, 3'-galactosyllactose, lacto- N-hexaose and lacto-N-neohexaose, sialyl-lacto-N-tetraose a, sialyllacto-N-tetraose b, sialyllacto-N-tetraose c, disialyllacto-N-tetraose, 3' and 6' sialyllactose, lacto-N-tetraose, difucosyllactose, lacto-N-neotetraose, fructooligosaccharides (FOS), galactooligosaccharides (GOS), and combinations thereof.

Proteins suitable for use in the infant formulas include hydrolyzed, partially hydrolyzed, and non-hydrolyzed or intact proteins or protein sources, and can be derived from any known or otherwise suitable source such as milk (e.g., casein, whey), animal (e.g., meat, fish), cereal (e.g., rice, corn), vegetable (e.g., soy), or combinations thereof. Proteins for use herein can also include,
or be entirely or partially replaced by, free amino acids known for or otherwise suitable for use in infant formulas, non-limiting examples of which include alanine, arginine, asparagine, carnitine, aspartic acid, cystine, glutamic acid, glutamine, glycine, histidine, isoleucine, leucine, lysine, methionine, phenylalanine, proline, serine, taurine, threonine, tryptophan, taurine, tyrosine, valine, and combinations thereof. These amino acids are most typically used in their L-forms, although the corresponding D-isomers may also be used when nutritionally equivalent. Racemic or isomeric mixtures may also be used.

Fats suitable for use in the infant formulas include coconut oil, soy oil, corn oil, olive oil, safflower oil, high oleic safflower oil, algal oil, MCT oil (medium chain triglycerides), sunflower oil, high oleic sunflower oil, palm and palm kernel oils, palm olein, canola oil, marine oils, cottonseed oils, and combinations thereof.

Vitamins and similar other ingredients suitable for use in the infant formulas of the present invention include vitamin A, vitamin D, vitamin E, vitamin K, thiamine, riboflavin, pyridoxine, vitamin B12, niacin, folic acid, pantothenic acid, biotin, vitamin C, choline, inositol, salts and derivatives thereof, and combinations thereof.

Minerals suitable for use in the infant formulas include calcium, phosphorus, magnesium, iron, zinc, manganese, copper, chromium, iodine, sodium, potassium, chloride, and combinations thereof. Preferably, the above nutrients are incorporated into the infant formulae consistent with accepted guidelines for the appropriate geographical and user demographic, for example, such as described in 21 U.S.C. §350a (2018).

The infant formulas or GUMs may further comprise other optional ingredients that may modify the physical, chemical, aesthetic or processing characteristics of the compositions or serve as pharmaceutical or additional nutritional components when used in the targeted infant population. Non-limiting examples of such optional ingredients include preservatives, additional antioxidants, emulsifying agents, buffers, colorants, flavors, nucleotides and nucleosides, probiotics, prebiotics, lactoferrin and related derivatives, thickening agents, and stabilizers, and so forth.

### Examples

The following Examples further detail and explain the performance of the inventive processes. Those skilled in the art will recognize many variations that are within the spirit of the invention and scope of the claims.

### Analytical method

Samples and standards were analyzed after proper dilution with High Performance Anion Exchange Chromatography (HPAEC) coupled to Pulsed Amperometric Detection (PAD). Samples and standards were injected (10 uL) on a Thermo Dionex ICS3000 system. Separation was carried out on a Thermo Dionex Carbopac PA1 column (250x4 mm) equipped with a Thermo Dionex Carbopac PA1 guard column (50x4 mm). Compounds of interest, i.e. lactitol, 2'-fucosyllactose (2'-FL), lactose and 2'-fucosyllactitol (2'-FLol), were eluted with 100 mM NaOH as mobile phase with a flow of 1.5 mL/min for 10 min. Quantification of lactitol, 2'-FL and lactose was carried out using external calibration with commercially available reference material. 2'-FLol was quantified by external calibration based on lactitol reference material, in absence of reference material of 2'-FLol, assuming the same response of lactitol and 2'-FLol with the PAD.

### Comparative examples

Several commercial 2'- fucosyllactose products were analyzed for the amount of 2'- fucosyllactitol present, using the analytical method as described, and compared to the materials of the present invention represented by Composition 1 and Composition 2.

**Table 1**

| **Sample** | **2'-fucosyllactitol (wt%)** |
|---|---|
| Composition 1 | 2.2 |
| Composition 2 | 3.86 |
| Friesland Campina | 0.19 |
| Dupont | not detected |
| Glycom | 0.01 |
| Jennewein Biotechnologie GmbH | 0.1 |

### Enzymatic hydrolysis of 2'-fucosyllactitol by beneficial gut bacteria

To simulate the efficient hydrolysis of 2'-fucosyllactitol by gut bacteria, releasing lactitol, in vitro enzymatic hydrolysis experiments were carried out in a total volume of 1 ml. Commercially available fucosidases from Bifidobacterium longum and Bifidobacterium species were applied at 12 µg/ml, for the hydrolysis of 49 mM of 2-FL (control, Care4U, Dupont) or 47 mM 2'-FL and 2 mM 2'-FLol (composition 2) present in 800 µl of 125 mM sodium phosphate buffer of pH 6.5, by adding 200 µl of enzyme solution for 24 hours at 37 C and 1000 rpm (in a thermomixer from Eppendorf).

**Table 2.**

| Enzyme Product code | Supplier | Description | Glycoside Hydrolase Family | EC Number | Species | Enzyme concentration (mg/ml) |
|---|---|---|---|---|---|---|
| CZ0511 | NZYtech | BlFuc95A | GH95 | 3.2.1.51 | *Bifidobacterium longum* | 1 |
| E-FUCM | Megazyme | 1,2-α-L-Fucosidase (microbial) | GH95 | 3.2.1.63 | *Bifidobacterium* sp. | 0.075 |

A sample of 125 µl was taken after 2 hours and immediately heated for 5 minutes at 95 C to inactivate the enzyme before analysis by HPAEC-PAD as described above.

**Table 3.**

| Substrate | Fucosidase | 2'-FL (wt%) | 2'-FLol (wt%) |
|---|---|---|---|
| Care4U | *Bifidobacterium longum* | 0 | not detected |
| Care4U | *Bifidobacterium* sp. | 3 | not detected |
| Care4U | None (control reaction) | 100 | not detected |
| Composition 2 of present invention | *Bifidobacterium longum* | 0 | 0 |
| Composition 2 of present invention | *Bifidobacterium* sp. | 18 | 14 |
| Composition 2 of present invention | None (control reaction) | 99 | 97 |

The data confirms that fucosidases typically present in the digestive tract of mammals can hydrolyze both 2'-FL as well as 2'-FLol.

### Enzymatic hydrolysis of 2'-fucosyllactitol releasing lactitol

To simulate the efficient hydrolysis of 2'-fucosyllactitol and release of lactitol by gut bacteria, in vitro enzymatic hydrolysis experiments were carried out using commercially available fucosidases from Bifidobacterium longum and Bifidobacterium species, applied at two different concentrations (2 and 12 µg/ml).

The hydrolysis of composition 2 (comprising 47 mM 2'-FL and 2 mM 2'-FLol) was carefully monitored for 24 hours at 37 C and 1000 rpm (in a thermomixer from Eppendorf). Composition 2 was dissolved in 125 mM sodium phosphate buffer of pH 6.5, and to 800 µl of this solution the enzymes were added at the defined concentrations. Time samples were taken after 10 mins, 30 mins, 2 hrs., 8 hrs. and 24 hrs. After taking the time sample, it was immediately heated for 5 minutes at 95 C to inactivate the enzyme before analysis by HPAEC-PAD as described above. The data was normalized towards the maximum amount and represented as relative amount in percentages, where the maximum amount in the time series was set at 100%.

**Table 4.**

| Fucosidase | Dosage (µg /ml) | Time (hours) | Lactitol | 2'-FLol | Lactose | 2'-FL |
|---|---|---|---|---|---|---|
| *Bifidobacterium longum* | 12 | 0.17 | 78% | 34% | 75% | 17% |
| | 12 | 0.50 | 98% | 7% | 87% | 1% |
| | 12 | 2.00 | 96% | 0% | 85% | 0% |
| | 12 | 8.00 | 100% | 0% | 85% | 0% |
| *Bifidobacterium* sp. | 12 | 0.17 | 22% | 79% | 30% | 83% |
| | 12 | 0.50 | 43% | 51% | 54% | 55% |
| | 12 | 2.00 | 83% | 11% | 90% | 4% |
| | 12 | 8.00 | 99% | 0% | 100% | 0% |
| None (control) | 0 | 0.17 | 0% | 92% | 2% | 98% |
| | 0 | 0.50 | 0% | 81% | 2% | 96% |
| | 0 | 2.00 | 0% | 92% | 2% | 95% |
| | 0 | 8.00 | 0% | 87% | 2% | 99% |
| *Bifidobacterium* sp. | 2 | 0.17 | 14% | 72% | 22% | 75% |
| | 2 | 0.50 | 26% | 69% | 34% | 66% |
| | 2 | 2.00 | 64% | 25% | 74% | 16% |
| | 2 | 8.00 | 87% | 0% | 87% | 0% |
| | 2 | 24.17 | 87% | 0% | 85% | 0% |
| *Bifidobacterium longum* | 2 | 0.17 | 21% | 69% | 26% | 66% |
| | 2 | 0.50 | 43% | 48% | 52% | 42% |
| | 2 | 2.00 | 76% | 12% | 82% | 3% |
| | 2 | 8.00 | 91% | 0% | 93% | 0% |
| | 2 | 24.17 | 86% | 0% | 87% | 0% |
| None (control) | 0 | 0.17 | 0% | 83% | 2% | 91% |
| | 0 | 0.50 | 0% | 90% | 2% | 99% |
| | 0 | 2.00 | 0% | 93% | 2% | 94% |
| | 0 | 8.00 | 0% | 100% | 2% | 100% |
| | 0 | 24.17 | 0% | 96% | 2% | 98% |

By incubating composition 2 with either 12 µg/ml Bifidobacterium longum fucosidase (Fig. 2), 12 µg/ml Bifidobacterium sp. fucosidase (Fig. 3), 2 µg/ml Bifidobacterium longum fucosidase (Fig. 4) or 2 µg/ml Bifidobacterium sp. fucosidase (Fig. 5), both 2'-FL and 2'-FLol comprising the composition of the present invention are readily hydrolyzed, releasing lactose and lactitol, respectively. In contrast, the control, where no enzyme was added, was stable, with no hydrolysis of either 2'-FL or 2'-FLol (Fig. 6). Thus, the data confirms that fucosidases typically present in the digestive tract of mammals can hydrolyze both 2'-FL as well as 2'-FLol, and thereby release lactose and lactitol, respectively.

Other features, advantages and embodiments of the invention disclosed herein will be readily apparent to those exercising ordinary skill after reading the foregoing disclosure. In this regard, while specific embodiments of the invention have been described in considerable detail, variations and modifications of these embodiments can be affected without departing from the spirit and scope of the invention as described and claimed.

## Claims

1. A prebiotic solids product comprising 2'-fucosyllactose and 0.2 to 6.0 wt.% of 2'-fucosyllactitol based on the total wt.% of the prebiotic solids product.

2. The prebiotic solids product of claim 1, wherein the 2'-fucosyllactitol is present in the prebiotic solids product in an amount of 0.5 to 6.0 wt.%.

3. The prebiotic solids product of claim 1 or 2, wherein the 2'-fucosyllactitol is present in the prebiotic solids product in an amount of 0.5 to 3.5 wt.%.

4. The prebiotic solids product of claim 1 or 2, wherein the 2'-fucosyllactitol is present in the prebiotic solids product in an amount of 2.0 to 4.5 wt.%.

5. The prebiotic solids product of any of claims 1, 2 or 4, wherein the 2'-fucosyllactitol is present in the prebiotic solids product in an amount of 2.0 to 4.0 wt.%.

6. The prebiotic solids product of any preceding claims, wherein the 2'-fucosyllactose is present in the prebiotic solids product in an amount from 86.0 to 94.0 wt.% based on the total wt.% of the prebiotic solids product.

7. The prebiotic solids product of claim 6, wherein the ratio of 2'-fucosyllactose/2'-fucosyllactitol in the prebiotic solids product is from 14.3 to 470.

8. The prebiotic solids product of any preceding claims, wherein the prebiotic solids product is a dried powder.

9. A process comprising administering an effective amount of the prebiotic solids product of any preceding claims to a subject in need thereof, thereby generating an *in vivo* supply of lactitol.

10. A process for making a food, growing up milk, dietary supplement or medicine, wherein the process comprises:
mixing the prebiotic solids product of any preceding claims with one or more ingredients suitable for the food, growing up milk, dietary supplement or medicine, or
dissolving the prebiotic solids product of any preceding claims in a solvent, preferably water, and mixing the dissolved prebiotic solids product with one or more ingredients suitable for making of the food, growing up milk, dietary supplement or medicine.

11. A process for making infant formula, wherein the process comprises:
mixing the prebiotic solids product of any preceding claims with at least one infant formula ingredient, or
dissolving the prebiotic solids product of any preceding claims in a solvent, preferably water, and mixing the dissolved prebiotic solids product with at least one infant formula ingredient.

12. A food, growing up milk, dietary supplement or medicine obtained from the process of claim 10.

13. An infant formula obtained from the process of claim 11.
